# EUROPEAN PATENT SPECIFICATION

(11) **EP 0 666 454 B1**
(45) Date of publication and mention of the grant of the patent: **12.09.2001**
(21) Application number: 94300911.8
(22) Date of filing: 08.02.1994
(51) Int. Cl.: F24F 5/00

(54) **An air conditioning/providing system directly through natural heat preserving main body**
Luft Klimatisier/Zufuhr-Anlage mit Naturwärmespeicher
Système de conditionnement/alimentation d'air et accumulateur de chaleur naturel

(43) Date of publication of application: 09.08.1995
(73) Proprietor: Yang, Tai-Her, Si-Hu Town, Dzan-Hwa (TW)
(72) Inventor: Yang, Tai-Her, Si-Hu Town, Dzan-Hwa (TW)
(74) Representative: Pratt, David Martin

(56) References cited:
- CH-A- 671 622
- US-A- 2 793 509
- US-A- 4 323 113
- PATENT ABSTRACTS OF JAPAN vol. 6, no. 36 (M-115) 5 March 1982 & JP-A-56 151 824 (MIYAUCHI HITOSHI) 25 November 1981

## Description

The present invention relates to an open-cycle system for supplying air to, and regulating the temperature of, a region.

Conventional room temperature regulating systems such as an air conditioner, for example, use a refrigerant compressor as the main basis for reducing the temperature. Conventional air-heaters use electric power or, alternatively, burn materials in order to produce heat, thereby wasting a lot of energy in order to condition the temperature of a room; they also produce a great deal of pollution from exhaust heat and materials. The more recent conditioners, for example, ice-preserving types, use off-peak electrical power to freeze water into ice, and then use the peak power to melt the ice through a temperature-preserving tube in order to achieve the required temperature. This type of ice-preserving air-conditioner, however, has many disadvantages, needing a substantial amount of space for a bulky freezer and a well-insulated refrigerator as well as requiring input energy. The present invention is able to use the surface and subsequent layers of the earth's surface, and also ponds, lakes and rivers as a naturally-occurring body in order to preserve temperature.

As a result of providing the room to be air conditioned with air from the natural body, the temperature and pressure of the air within the room will be similar to that of the natural body. As a result, the positive pressure will reduce the dust that has settled on doors and windows, while keeping the air free from pollution.

In the event of a city being situated in a basin, the resultant positive pressure will have the effect of dispersing the new uncontaminated air through convection.

Tests have shown that, in winter and in summer, at depths of 10-50 metres, the surface layers of the earth are seldom affected by the temperature on the earth's surface due to its heat capacity being very large. Similarly, sea water, lakes and rivers on the earth's surface are also able to preserve a constant temperature below a certain depth. In the description of the invention, these parts of earth's surface will be referred to as a "naturally-occurring body". Due to the fact that the naturally-occurring body has a large heat capacity, its temperature is always lower than that of the air on the surface of the earth during the summer; the river water temperature is usually below 15°C. In winter however, and in particular in areas of high latitude, where the earth's surface temperature is often between -20°C and -30°C, the naturally-occurring body will not decrease below 0°C (1 to 7°C in deep sea). Therefore, when a tube is connected between the naturally-occurring body and a house, in order to transfer fresh air and regulate the temperature, it will work in the same way as an air conditioner. This will have major benefits over conventional air conditioners, most notably reduced costs and exhaust energy.

US Patent No. 4,323,113 and US Patent No 2,793,509 both disclose underground air tempering systems for warming the supply air to a building in the winter and cooling the supply air in the summer in which a heat exchanger is disposed beneath the surface of the ground.

The present invention provides an open-cycle system for supplying air to , and regulating the temperature of, a region, the system comprising
a) a heat exchanger located in a naturally occurring body of substantially constant temperature, the heat exchanger having an inlet and an outlet;
b) a first conduit connected to the inlet of the heat exchanger so as to direct air into the heat exchanger such that the air assumes a temperature substantially equal to that of the naturally occurring body, the air taken from ambient atmosphere externally of said region.
c) at least one second conduit connected to the outlet of the heat exchanger so as to direct the air through at least two outlets into said region; and
d) pump means connected to the first and each second conduit so as to direct the air through the first conduit, the heat exchanger and the second conduit, into said region.

The air is circulated through the heat exchanger by a main pump, or further by a shunt pump, wherein the fresh air enters the system through a filtered inlet, whereupon it is circulated through a heat exchanger within the naturally-occurring body after which it is pumped through a transfer tube and into the room that is to be heated. If the conductive tube is long enough to be buried in the naturally-occurring body, and the tube is made of good conductive materials so as to actuate the function of a heat exchanger, the heat exchanger itself can be omitted.

A filter, such as active carbon filter, can be attached to the inlet and the outlet of the system, together with means for testing and regulating gaseous emissions.

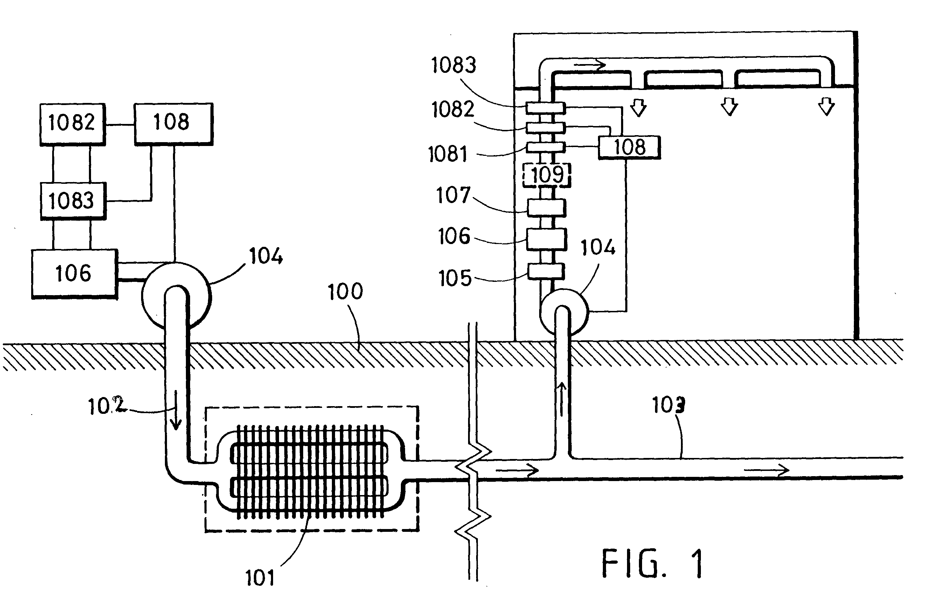

The present invention will now be described by way of example, with reference to the accompanying drawing, wherein Figure 1 is a schematic view of the present invention.

With reference to the figure, the present invention comprises a heat exchanger 101 situated within a naturally-occurring body 100 of substantially constant temperature, the heat exchanger being made of conductive material and consisting of a heating tube, an inlet and an outlet such that air is transmitted from the inlet through the heat exchanger, to the outlet, while retaining a similar temperature to that of the naturally-occurring body 100. The naturally-occurring body 100 comprises solid or liquid mediums having large, stable, heat capacities compared with their surroundings. These may include the earth's surface or layers beneath the surface, ponds, lakes, rivers, deserts, icebergs, and oceans.

A first transmitting pipe line 102 is connected to the heat exchanger, and passes through the naturally-occurring body so as to transmit fresh air through it, the fresh air reaching the same temperature as the body 100. The first pipe line 102 is made of good conductive materials when it is situated at great depth so as to be able to regulate the temperature of the fresh air. When the first pipe line is exposed to the open air, it is wrapped with, or made of, insulated materials.

A second pipe line 103 transmits the fresh air at a similar temperature to that of the naturally-occurring body 100, to the room to be air conditioned.

An air pump 104 driven by electrical or mechanical power is connected to the second pipe line 103 in order to circulate the air through the pipe lines. The air pump 104 can be used to pump the air current in and out by means of the temperature difference of the troposphere.

A flow gauge 105 can be included to calculate the flow of fresh air, so that the value can be referred to when the subscribers have to pay the charge.

An air filter 106 is included to prevent the pipe line from being worn through longterm use. The filter 106 is situated at the air inlet and outlet. It can include a dust filtering net, a harmful air filter, and/or an active carbon filter.

A flow regulating valve 107 is included to regulate the air flow. The valve 107 can be controlled manually or mechanically.

An auxiliary regulating relay 109 is included if the heat exchanger is unable to bring the air to the required temperature. The relay 109 includes heating devices to increase the temperature of the air by either using the temperature of the naturally-occurring body, or by other conventional techniques such as burning, electrical heat, or solar energy. The regulating relay 109 can also reduce the temperature by conventional cooling techniques.

A control unit 108 is connected to harmful air detectors 1081 situated at the inlet /outlet port. In the event of harmful air entering the system the control unit is able to shut down the system by for example cutting off the air supply and providing a warning siren. The harmful air detector 1081 is positioned at the side of the input/output port to detect the harmful air in the room. The control unit 108 further includes a temperature monitoring device 1082, and a fresh air flow gauge and indicator 1083.

In addition to being the source of fresh air for preserving the temperature, the system can regulate the temperature within the room. For example, in winter, when the room temperature needs to be higher than the temperature of the naturally-occurring body 108, the system will have a pre-heating function.

Likewise, in summer, when the room temperature needs to be lower than the temperature of the naturally-occurring body 100, but the temperature of the fresh air is lower than the environmental temperature outside the room, the system will also have a pre-cooling function.

The air output of the system has a positive pressure between the surrounding environment and space. Therefore, the system can release fresh air due to the positive pressure being built up between indoors and outdoors, making it harder for outdoor floating dust and polluted air to enter the room. Also, in relation to a whole city, an air current source of positive pressure will be built up to disperse outwardly thus improving the retaining effect of a high sphere air current built up by the land form of a basin.

The heat exchanger can be suspended above, or within, oceans, lakes, ponds, rivers, or artificial pools, which are to be used as the naturally-occurring body 100 to provide fresh air at substantially constant temperature for the cabin of a ship and other equipment as well as buildings on the ground.

As well as providing a healthier atmosphere. the system is relative inexpensive, only requiring the cost of the primary equipment initially and the energy for the pumps later on. The system also provides a high energy saving, especially when it uses the method of natural convection, where exhaust energy is minimal.

## Claims

1. An open-cycle system for supplying air to , and regulating the temperature of, a region, the system comprising
- a heat exchanger (101) located in a naturally occurring body (100) of substantially constant temperature, the heat exchanger having an inlet and an outlet;
- a first conduit (102) connected to the inlet of the heat exchanger (101) so as to direct air into the heat exchanger (101) such that the air assumes a temperature substantially equal to that of the naturally occurring body (100), the air taken from ambient atmosphere externally of said region;
**characterised in that** the system further comprises
- at least one second conduit (103) connected to the outlet of the heat exchanger (101) so as to direct the air through at least two outlets into said region; and
- pump means (104) connected to the first and each second conduit so as to direct the air through the first conduit (102), the heat exchanger (101) and the second conduit (103), into said region.

2. A system as claimed in claim 1, wherein the air is capable of maintaining a substantially constant temperature.

3. A system as claimed in claim 1 or claim 2, further comprising an air filter (106) connected to the pump means (104).

4. A system as claimed in any one of claims 1 to 3, further comprising a control unit (108) connected to the pump means (104).

5. A system as claimed in claim 4, further comprising:
a) a temperature sensor (1082) connected to the pump means (104) and a control unit (108);
b) a flow gauge (1083) connected to the pump means (104) and to the control unit (108); and
c) a hazardous gas detection device (1081) connected to the pump means (108) and the control unit (108).

6. A system as claimed in any one of claims 1 to 5, further comprising a flow regulating valve (107) to control the flow of air into said region.

7. A system as claimed in any one of claims 1 to 6, wherein said region includes a semi-closed space with an exhaust opening or an open space.

## Patentansprüche

1. Luft-Klimatisier/Zufuhr-Anlage zum Klimatisieren bzw. Regeln der Temperatur eines Bereichs mit
- einem Wärmetauscher (101), der in einer natürlich vorkommenden Masse (100) im wesentlichen konstanter Temperatur angeordnet ist und einen Zu- und einen Ablauf aufweist, und
- einer ersten Leitung (102), die mit dem Zulauf des Wärmetauschers (101) verbunden ist, um Luft so in den Wärmetauscher (101) zu richten, dass diese eine Temperatur im wesentlichen gleich der der natürlich vorkommenden Masse (100) annimmt, wobei die Luft der umgebenden Atmosphäre außerhalb des Bereichs entnommen wird, weiterhin **gekennzeichnet durch**
- mindestens eine zweite Leitung (103), die mit dem Ablauf des Wärmetauschers (101) verbunden ist, um die Luft durch mindestens zwei Abläufe in den Bereich zu führen, und
- eine Pumpeinrichtung (104), die mit den ersten und beiden zweiten Leitungen verbunden ist, um die Luft durch die erste Leitung (102), den Wärmetauscher (101) und die zweite Leitung (103) in den Bereich zu richten.

2. System nach Anspruch 1, bei dem die Luft eine im wesentlichen konstante Temperatur beibehalten kann.

3. System nach Anspruch 1 oder Anspruch 2, weiterhin mit einem Luftfilter (106), das mit der Pumpeinrichtung (104) verbunden ist.

4. System nach einem der Ansprüche 1 bis 3, weiterhin mit einer Steuereinheit (108), die mit der Pumpeinrichtung (104) verbunden ist.

5. System nach Anspruch 4 weiterhin mit
a. einem Temperaturfühler (1082), der mit der Pumpeinrichtung (104) und einer Steuereinheit (108) verbunden ist,
b. einem Strömungsmessgerät (1083), das mit der Pumpeinrichtung (104) und der Steuereinheit (108) verbunden ist, und
c. einer gefährliche Gase erfassenden Einrichtung (1081), die mit der Pumpeinrichtung (104) und der Steuereinheit (108) verbunden ist.

6. System nach einem der Ansprüche 1 bis 5 weiterhin mit einem Strömungseinstellventil (107) zum Steuern der Luftzufuhr zum Bereich.

7. System nach einem der Ansprüche 1 bis 6, bei dem der Bereich einen halbgeschlossenen Raum mit einer Abluftöffnung oder einen offenen Raum aufweist.

## Revendications

1. Système à cycle ouvert d'alimentation en air et de régulation de la température de l'air d'une zone, le système comprenant :
. un échangeur de chaleur (101) situé dans un corps naturel (100) de température sensiblement constante, l'échangeur de chaleur présentant une entrée et une sortie ;
. un premier conduit (102) connecté à l'entrée de l'échangeur de chaleur (101) de manière à diriger l'air à l'intérieur de l'échangeur de chaleur (101) de façon que l'air prenne une température sensiblement égale à celle du corps naturel (100), l'air étant pris dans l'atmosphère ambiante externe à la zone ;
**caractérisé en ce que** le système comporte en outre :
. au moins un second conduit (103) connecté à la sortie de l'échangeur de chaleur (101) de manière à diriger l'air à travers au moins deux sorties dans cette zone ; et
. des moyens de pompage (104) connectés au premier et à chaque second conduit de manière à diriger l'air à travers le premier conduit (102), l'échangeur de chaleur (101) et le second conduit (103), à l'intérieur de cette zone.

2. Système selon la revendication 1, **caractérisé en ce que** l'air est capable de maintenir une température sensiblement constante.

3. Système selon la revendication 1 ou la revendication 2, **caractérisé en ce qu'**il comprend en outre un filtre à air (106) relié aux moyens de pompage (104).

4. Système selon l'une quelconque des revendications 1 à 3, **caractérisé en ce qu'**il comprend en outre une unité de régulation (108) reliée aux moyens de pompage (104).

5. Système selon la revendication 4,
**caractérisé en ce qu'**il comprend en outre :
a) une sonde de température (1082) connectée aux moyens de pompage (104) et à l'unité de régulation (108) ;
b) un débitmètre (1083) relié aux moyens de pompage (104) et à l'unité de régulation (108) ; et
c) un dispositif de détection de gaz dangereux (1081) relié aux moyens de pompage (104) et à l'unité de régulation (108).

6. Système selon l'une quelconque des revendications 1 à 5, **caractérisé en ce qu'**il comprend en outre une soupape de régulation (107) pour réguler le débit d'air dans cette zone.

7. Système selon l'une quelconque des revendications 1 à 6, **caractérisé en ce que** cette zone comprend un espace semi-fermé avec une ouverture d'échappement ou un espace ouvert.
